# EUROPÄISCHE PATENTANMELDUNG

(11) **EP 1 555 528 A1**
(43) Veröffentlichungstag der Anmeldung: **20.07.2005**
(21) Anmeldenummer: 04000721.3
(22) Anmeldetag: 15.01.2004
(51) Int. Cl.: G01N 33/50, G01N 33/68

(54) **Verfahren zur Untersuchung der Aktivität von Ionenkanälen**

(71) Anmelder: Evotec OAI AG, 22525 Hamburg (DE)
(72) Erfinder: Ebneth, Andreas, 25421 Pinneberg (DE); Netzer, Rainer, 22549 Hamburg (DE); Hahn, Ulrike, 21465 Reinbek (DE)
(74) Vertreter: Meyers, Hans-Wilhelm, Dr.Dipl.-Chem.

(57) **Zusammenfassung**

Die vorliegende Erfindung betrifft ein Verfahren zur Untersuchung der Aktivität von lonenkanälen mit folgenden Schritten:
- Bereitstellen einer Probe enthaltend eine oder mehrere Zellen, welche membranständige lonenkanäle aufweisen, und
- Ermitteln eines Wertes eines Messparameters als Indikator für die Aktivität der lonenkanäle,
dadurch gekennzeichnet, dass die Ermittlung des Wertes des Messparameters bei einer Temperatur ≤ etwa 10 °C durchgeführt wird.

## Beschreibung

Die Membranen lebender Zellen erfüllen eine Vielzahl von Funktionen, die für die Unversehrtheit und Aktivität von Zellen und Geweben von großer Bedeutung sind. Abgrenzung und Regelung des Zellinhaltes, Stoffaustausch sowie Weiterleitung von Signalen sind Beispiele für derartige Funktionen. Geladene Moleküle und anorganische lonen (wie beispielsweise Na⁺-, K⁺, Ca²⁺- und CI⁻-Ionen) können Membranen nicht durch einfache Diffusion durch die Lipiddoppelschicht durchqueren, sondern benötigen hierzu bestimmte Transportsysteme der Membran. Derartige Transportsysteme umfassen insbesondere lonenkanäle, von denen eine große Vielzahl nicht zuletzt aufgrund ihrer immensen Bedeutung für diverse Krankheitsbilder sehr gut u.a. im Hinblick auf ihre biochemischen und elektrophysiologischen Eigenschaften charakterisiert sind. Derartige lonenkanäle können gezielt geöffnet und geschlossen werden, so dass lonen nicht ständig durch diese fließen können. Der Nettodurchfluss von individuellen lonen wird hierbei von Faktoren wie der Permeabilität für dieses lon, dem Konzentrationsgradienten des lons und der elektrischen Potentialdifferenz zwischen den beiden Seiten der Membran bestimmt. Im allgemeinen unterscheidet man Ionenkanaltypen, die auf eine Änderung des elektrischen Potentials reagieren (spannungsabhängige lonenkanäle) von solchen, die auf bestimmte Signalstoffe, sog. Transmitter, reagieren. Des weiteren sind lonenpumpen bekannt, die für den aktiven lonentransport entgegen des elektro-chemischen Gradienten unter Energieverbrauch sorgen. Auf diese Weise werden charakteristische Unterschiede in den Ionenkonzentrationen zwischen intra- und extrazellulärem Raum aufgebaut bzw. aufrechterhalten. Ein wichtiges Beispiel ist die sogenannte Natrium-Kalium-Pumpe, welche einen gekoppelten Transport von Natrium und Kalium unter Verbrauch von ATP als Energiequelle ermöglicht.

Es sind eine Vielzahl von Krankheitsbildern bekannt, die medikamentös durch eine gezielte Beeinflussung der Aktivität von Ionenkanälen behandelt werden. Hierzu gehören u.a. Antiarrythmika, d.h. Mittel zur Behandlung von Herzrhythmusstörungen, die nach ihren elektrophysiologischen Wirkungsmechanismen in unterschiedliche Klassen eingeteilt werden. So wirkt beispielsweise der Calciumantagonist Verapamil über eine Blockade von Calciumkanälen, währenddessen Vertreter der Kaliumantaganisten wie Amiodaron und Sotalol durch Blockade von Kaliumkanälen eine selektive Verlängerung der Aktionspotentialdauer bewirken. Weitere Krankheitsbilder, die durch eine Aktivierung oder Blockade von lonenkanälen beeinflußt werden können, sind z.B. eine große Anzahl der ZNS-Erkrankungen (Epilepsie, Schmerz, Schlaganfall, Migräne), Autoimmunerkrankungen, Krebs oder Diabetes.

Im Rahmen der pharmazeutischen Wirkstoffforschung ist es wünschenswert, Testverfahren zur Verfügung zu haben, mit denen der Einfluss einer potentiell pharmakologisch aktiven Substanz auf derartige Ionenkanäle genau erfasst werden kann. Dies ist zum einen von Bedeutung für die Entwicklung von Substanzen, deren Wirkmechanismus auf der gezielten Beeinflussung von lonenkanälen basiert, zum anderen aber auch für die Evaluierung von potentiellen Nebenwirkungen, d.h. die unerwünschte Beeinflussung von lonenkanalaktivitäten durch die putativen Pharmaka.

Das Membranpotential lebender Zellen wird maßgeblich durch die intra- und extrazellulären Natrium-, Kalium- und Chlorid-lonenkonzentrationen bestimmt. Untersucht man beispielsweise den Einfluss der Blockierung eines Kaliumkanals auf das Ruhemembranpotential lebender Zellen, dann wird nach der Goldmann-Hodgin-Katz-Gleichung im wesentlichen die Leitfähigkeit von Kalium über die Membran verändert, was sich auf das Membranpotential auswirkt. Dieser Veränderung können Zellen unter anderem damit begegnen, dass die Leitfähigkeit anderer lonen über die Membran verändert wird, so dass ein Nettoeinfluss der Kaliumkanalblockade auf das Membranpotential verringert oder sogar verhindert wird. Dies kann durch die Aktivierung von Pumpensystemen in den Zellen erfolgen, die aktiv lonen transportieren.

Erfasst man nunmehr in einem Testverfahren das Membranpotential lebender Zellen unter Einfluss einer potentiellen oder bekannten pharmakologisch aktiven Substanz, so besteht die Gefahr, dass der gemessene Potentialwert aufgrund der beschriebenen Gegenregulationsmechanismen verfälscht ist. Diese Verfälschung kann sogar soweit gehen, dass eine Beeinflussung des Membranpotentials bei Vorliegen eines ungünstigen Signal-Rausch-Verhältnisses gegebenenfalls nicht mehr erkennbar ist.

Aufgabe der vorliegenden Erfindung ist es somit, ein Testverfahren zur Untersuchung der Aktivität von lonenkanälen bereitzustellen, welches die vorgenannten Störeinflüsse minimiert.

Diese Aufgabe wird gelöst durch ein Verfahren mit den Merkmalen des unabhängigen Anspruchs 1. Die weiteren Ansprüche betreffen bevorzugte Ausführungsformen der vorliegenden Erfindung.

Die vorliegende Erfindung betrifft ein Verfahren zur Untersuchung der Aktivität von lonenkanälen mit folgenden Schritten:
- Bereitstellen einer Probe enthaltend eine oder mehrere Zellen, welche membranständige lonenkanäle aufweisen, und
- Ermitteln eines Wertes eines Messparameters als Indikator für die Aktivität der lonenkanäle,
dadurch gekennzeichnet, dass die Ermittlung des Wertes des Messparameters bei einer Temperatur deutlich geringer als die übliche Raumtemperatur, insbesondere ≤ etwa 10 °C, durchgeführt wird.

Die vorliegende Erfindung beruht auf der Erkenntnis, dass man durch Temperaturerniedrigung den Zellen die Möglichkeit entziehen kann, das Membranpotential durch die Aktivierung der vorgenannten Pumpensysteme zu beeinflussen. Im Stand der Technik beschriebene Testverfahren z.B. zur Evaluierung von Kaliumkanalblockern werden typischerweise bei Körpertemperatur, d.h. 37 °C, oder bei Raumtemperatur durchgeführt. Entzieht man den Zellen die Möglichkeit, das Membranpotential durch die Aktivierung der vorgenannten Pumpensysteme zu beeinflussen, indem man die Temperatur erniedrigt, dann können die Zellen einer Änderung des Membranpotentials durch Blockade von Kalium- oder Natriumkanälen nicht mehr so effektiv wie bei 37 °C oder Raumtemperatur begegnen.

Gemäß der vorliegenden Erfindung ist es bevorzugt, eine Bestimmung des Messparameters bei Temperaturen von etwa ≤ 10 °C, insbesondere jedoch etwa ≤ 5 °C durchzuführen. Besonders bevorzugt sind hierbei Temperaturen etwa ≤ 2 °C. Die untere Temperaturgrenze beträgt vorzugsweise 0 °C. Da die zu untersuchenden Zellproben typischerweise in isotonischen Pufferlösungen vorliegen, ist es prinzipiell auch möglich, knapp unter 0 °C zu messen, typischerweise bis zu - 2 °C oder - 4 °C.

Vorzugsweise ist der Messparameter das Membranpotential der Zelle oder ein Maß hierfür. Es ist jedoch in einer weiteren Ausführungsform des erfindungsgemäßen Verfahrens auch möglich, dass der Messparameter eine extra- und/oder intrazelluläre lonenkonzentration oder ein Maß hierfür ist.

Insbesondere ist es bevorzugt, dass der Wert des Messparameters vor, während und/oder nach Zusatz einer die Aktivität der lonenkanäle (potentiell) beeinflussenden Testsubstanz ermittelt wird. Hierbei kann insbesondere die Aktivität eines transmitterabhängigen lonenkanals untersucht werden. Es kann jedoch auch die Aktivität eines spannungssensitiven lonenkanals ermittelt werden. Hierbei kann es sich insbesondere um die Aktivität eines Kaliumkanals, eines Natriumkanals oder eines Calciumkanals handeln.

Die Ermittlung eines Maßes für die lonenkonzentration oder das Membranpotential kann z.B. durch Fluoreszenzmethoden, radioaktive Methoden oder Atomabsorptionsspektroskopie erfolgen.

Als Maß für das Membranpotential kann z.B. die Fluoreszenzemission eines spannungssensitiven Fluoreszenzfarbstoffes, insbesondere des Farbstoffes Dibac4(3) dienen, wie weiter unten ausführlicher dargestellt. Es kann jedoch auch bevorzugt sein, die lonenkonzentration von Rubidium (als Austauschion), insbesondere radioaktivem Rubidium, zu messen. Ferner kann z.B. die Ionenkonzentration von Calcium mittels Chelatoren gemessen werden.

Die bei den erfindungsgemäß niedrigen Temperaturen durchzuführende Bestimmung des Membranpotentials kann insbesondere mittels an sich bekannter Fluoreszenzassays unter Verwendung handelsüblicher Fluoreszenzreader (z.B. FLIPR der Fa. Molecular Devices), konfokaler Fluoreszenzmikroskope oder durchflußcytometrischer Apparaturen durchgeführt werden. Hierbei können typischerweise potentialsensitive Fluoreszenzfarbstoffe, wie z.B. der handelsübliche Verteilungsfarbstoff Bis-(1,3-dibutylbarbitricacid)trimethaneoxonol (Dibac₄(3)), Anwendung finden. Dibac₄(3) ist ein Farbstoff vom Bis-oxonol-Typus, dessen Verteilung im Zellcytosol bei Membrandepolarisation erhöht wird. Dieser Vorgang geht einher mit einer Erhöhung der Fluoreszenzintensität. Tritt der Farbstoff somit bei einer Depolarisation des Ruhemembranpotentials der Zellen z. B. aufgrund einer Blockade von spannungsabhängigen Kaliumkanälen in die Zellen ein, so ist eine Erhöhung der Fluoreszenzintensität detektierbar. In vorteilhafter Weise wird im übrigen die Quantenausbeute dieses Oxonolderivats durch die erniedrigten Temperaturen begünstigt. Durch die hiermit einhergehende Erhöhung der Signalstärke kann somit zusätzlich zu der oben beschriebenen Inhibierung der zellulären Pumpsysteme ein nochmals verbessertes Signal-Rausch-Verhältnis herbeigeführt werden.

Ein weiterer Vorteil betrifft die Stabilität des auszulesenden Signals. Bei einer Durchführung des Tests bei 37 °C oder Raumtemperatur können die anfangs durch Zugabe eines Kanalblockers (wie z. B, Kaliumkanalblocker) auf Zellen hervorgerufenen Fluoreszenzsignale (z. B. Dibac₄(3)-Fluoreszenz) u.a. aufgrund der Tätigkeit von endogenen lonenpumpen nach einem kurzen transienten Anstieg wieder auf den Anfangszustand zurückgeführt werden. Dies bedeutet, daß das zeitliche Fenster zur Messung eines Effektes von Substanzen auf die lonenkanäle eng ist, so daß eine online-Messung in einem sehr kurzen Zeitfenster (typischerweise weniger als 2 min) nach Zugabe der Testsubstanz erfolgen muß, was mit hohen Anforderungen an das Messgerät verbunden ist. Wird der Test bei erniedrigten Temperaturen durchgeführt, dann beobachtet man einen solchen Abfall des initial hervorgerufenen Signals nicht oder nur in einem sehr verringerten Ausmaß. Dies hat den Vorteil, daß die Messung eines Effektes einer Substanz auf einen zu untersuchenden lonenkanal auch nach mehrstündiger Inkubation erfolgen kann. Dies wiederum erleichtert ein Screening einer großen Anzahl von Substanzen erheblich und erhöht damit den Durchsatz. Die Umwandlung eines transienten Signals in einen stabilen Ausleseparameter macht viele lonenkanäle erst für ein Screening zugänglich.

Die vorliegende Erfindung wird nachfolgend in einem experimentellen Beispiel verdeutlicht.

### Beispiel 1:

CHO Zellen, die stabil mit dem spannungsabhängigen Kaliumkanal HERG transfiziert wurden, wurden trypsiniert und abzentrifugiert. Die Zellen wurden danach in 1X Puffer (10 mM HEPES, pH 7,3, 140 mM Na⁺, 2 mM K⁺, 1 mM MgCl₂, 2 mM CaCl₂) mit 4µM DiBAC4(3) (Molecular Probes) aufgenommen und in einer Zahl von 2E4/well in 50 µl auf eine 384-well Mikrotiterplatte mit transparentem Boden gegeben, in der die folgenden Substanzen vorgelegt waren: 5 µl Puffer (2 mM K⁺), 5µl Puffer + 300 mM K⁺, und 5 µl Puffer + 10 µM E4031/2 mM K⁺.

Dibac₄(3) diente als spannungsabhängiger Fluoreszenzfarbstoff, der bei einer Depolarisierung der Zellmembran (z.B. Hervorgerufen durch Erhöhung der extrazellulären Kaliumkonzentration oder durch Blockade von Kaliumkanälen) durch die Zellmembran in die Zelle eintritt, dort an intrazelluläre Proteine und Membranen bindet, was zu einer Erhöhung der Fluoreszenz führt.

In den oben beschriebenen Ansätzen wurde die Kaliumkonzentration durch Zugabe einer geeigneten Stammmlösung auf 2 mM (Null-Kontrolle) sowie auf 30 mM gebracht (Kontrolldepolarisierung). Als Antagonist des HERG-Kaliumkanals wurde E4031 bei einer Kaliumkonzentration von 2 mM dazugegeben.

Die Auslesung erfolgte nach einer 150-minütigen Inkubation auf Eis sowie einer sich daran anschließenden Inkubation für 30 Minuten bei Raumtemperatur auf einem handelsüblichen Fluoreszenzlesegerät (Fluostar, bmg) bei einer Anregungswellenlänge von 485 nm und einer Emissionswellenlänge von 525 nm.

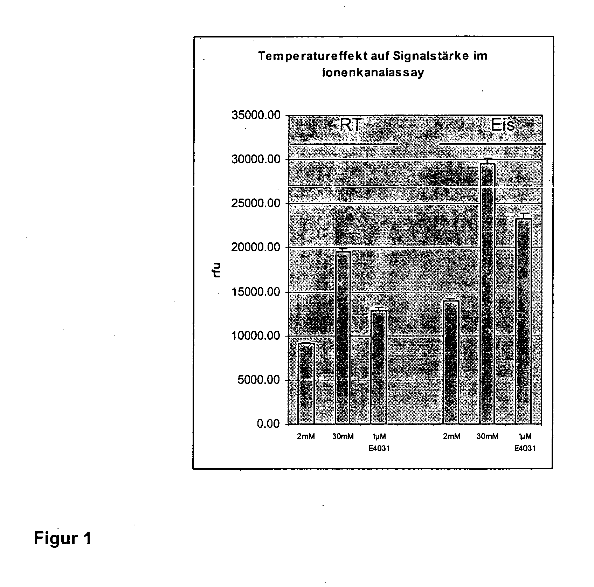

Die Ergebnisse des vorbeschriebenen Testverfahrens sind in nachstehender Tabelle 1 sowie Figur 1 zusammengefasst. Man kann erkennen, dass die Fluoreszenzsignalsteigerung, die durch Blockade des HERG-Kaliumkanals durch den Antagonisten E4031 hervorgerufen worden ist, bei Inkubation auf Eis signifikant stärker ist als bei Raumtemperatur. Die Steigerung des Signals erhöht sich von 41,76 % bei Raumtemperatur auf 66,62 % auf Eis.

**Tabelle 1**

| **150 min Inkubation auf Eis plus 30 min anschließende Inkubation bei Raumtemperatur** | | | | |
|---|---|---|---|---|
| | Mittelwert Rfu* | Stdabw ** | Fehler [%] | Signalsteigerung [%] *** |
| 2 mM K⁺ | 9037.67 | 211.27 | 2.34 | |
| 30 mM K⁺ | 19496.33 | 376.26 | 1.93 | 115.72 |
| 1µM | 12811.67 | 350.54 | 2.74 | 41.76 |
| E4031 | | | | |

| **150 min Inkubation auf Eis** | | | | |
|---|---|---|---|---|
| 2mM | 13997.67 | 217.94 | 1.56 | |
| 30mM | 29495.67 | 648.56 | 2.20 | 110.72 |
| 1µM | 23323.00 | 567.56 | 2.43 | 66.62 |
| E4031 | | | | |

| | | | | |
|---|---|---|---|---|
| * Rfu: relative Fluoreszenzintensität | | | | |
| ** Stdabw: Standardabweichung | | | | |
| *** Steigerung des ermittelten Fluoreszenzsignals im Vergleich zur Nullkontrolle (Fluoreszenzsignal bei 2 mM K⁺) | | | | |

## Patentansprüche

1. Verfahren zur Untersuchung der Aktivität von lonenkanälen mit folgenden Schritten:
- Bereitstellen einer Probe enthaltend eine oder mehrere Zellen, welche membranständige lonenkanäle aufweisen, und
- Ermitteln eines Wertes eines Messparameters als Indikator für die Aktivität der lonenkanäle,
**dadurch gekennzeichnet, dass** die Ermittlung des Wertes des Messparameters bei einer Temperatur ≤ etwa 10 °C durchgeführt wird.

2. Verfahren nach Anspruch 1, **dadurch gekennzeichnet, dass** die Ermittlung des Wertes des Messparameters bei einer Temperatur ≤ etwa 5 °C, insbesondere ≤ 2 °C, besonders bevorzugt zwischen 1 °C und 0 °C erfolgt.

3. Verfahren nach Anspruch 1 oder 2, **dadurch gekennzeichnet, dass** die Ermittlung des Wertes des Messparameters bei einer Temperatur zwischen etwa 10 °C und - 4 °C erfolgt.

4. Verfahren nach einem der vorhergehenden Ansprüche, **dadurch gekennzeichnet, dass** der Messparameter das Membranpotential der Zelle oder ein Maß hierfür ist.

5. Verfahren nach einem der vorhergehenden Ansprüche, **dadurch gekennzeichnet, dass** der Messparameter eine extra- und/oder intrazelluläre ionenkonzentration oder ein Maß hierfür ist.

6. Verfahren nach einem der vorhergehenden Ansprüche, **dadurch gekennzeichnet, dass** der Wert des Messparameters vor, während und/oder nach Zusatz einer die Aktivität der lonenkanäle potentiell beeinflussenden Testsubstanz ermittelt wird.

7. Verfahren nach einem der vorhergehenden Ansprüche, **dadurch gekennzeichnet, dass** die Aktivität eines transmitterabhängigen lonenkanals untersucht wird.

8. Verfahren nach einem der vorhergehenden Ansprüche, **dadurch gekennzeichnet, dass** die Aktivität eines spannungssensitiven Ionenkanals untersucht wird.

9. Verfahren nach einem der vorhergehenden Ansprüche, **dadurch gekennzeichnet, dass** die Aktivität eines Kaliumkanals, eines Natriumkanals oder eines Calciumkanals untersucht wird.

10. Verfahren nach einem der vorhergehenden Ansprüche, **dadurch gekennzeichnet, dass** die Ermittlung eines Maßes für die lonenkonzentration oder für das Membranpotential durch Fluoreszenzmethoden, radioaktive Methoden oder Atomabsorptionsspektroskopie erfolgt.

11. Verfahren nach einem der vorhergehenden Ansprüche, **dadurch gekennzeichnet, dass** als Maß für das Membranpotential die Fluoreszenzemission eines spannungssensitiven Fluoreszenzfarbstoffes, insbesondere des Farbstoffes Dibac4(3) dient.

12. Verfahren nach einem der vorhergehenden Ansprüche, **dadurch gekennzeichnet, dass** die Ionenkonzentration von Rubidium, insbesondere radioaktivem Rubidium, gemessen wird.

13. Verfahren nach einem der vorhergehenden Ansprüche, **dadurch gekennzeichnet, dass** die Ionenkonzentration, insbesondere von Calcium, mittels Chelatoren gemessen wird.

14. Verfahren nach einem der vorhergehenden Ansprüche, **dadurch gekennzeichnet, dass** die Werte mehrerer Messparameter ermittelt werden.

15. Verfahren nach einem der vorhergehenden Ansprüche zur Anwendung in der pharmazeutischen Wirkstoffforschung, insbesondere im Hochdurchsatzscreening von potentiell oder nachweislich aktiven pharmazeutischen Substanzen.
